# FASCICULE DE BREVET EUROPEEN

(11) **EP 2 608 813 B1**
(45) Date de publication et mention de la délivrance du brevet: **06.08.2014**
(21) Numéro de dépôt: 11708899.7
(22) Date de dépôt: 28.01.2011
(51) Int. Cl.: A61L 15/58

(54) **ARTICLE ADHÉSIF SUR LA PEAU**
AN DER HAUT ANHAFTENDER ARTIKEL
ARTICLE ADHESIVE TO THE SKIN

(30) Priorité: 01.02.2010 FR 1000388
(43) Date de publication de la demande: 03.07.2013
(73) Titulaire: Bluestar Silicones France, 69003 Lyon (FR)
(72) Inventeur: LORENTZ, Gilles, 69006 Lyon (FR); MARTIN, François, 23552 Lübeck (DE)
(74) Mandataire: Mekki, Boualem
(86) Numéro de dépôt international: PCT/FR2011/000056
(87) Numéro de publication internationale: WO 2011/092404

(56) Documents cités:
- WO-A1-2006/028612
- US-A1- 2007 042 108

## Description

Le domaine de l'invention est de celui des articles adhésifs à la peau pour un usage médical ou paramédical.

Il est très difficile de faire adhérer des matériaux aux films plastiques de basse énergie de surface tels que des films en polyester ou polyuréthane. L'art antérieur a décrit abondamment les primaires d'adhérence des gels silicones sur les films plastiques de basse énergie de surface. Typiquement les solutions de polymères du methylhydrogène siloxane permettent d'obtenir une adhésion satisfaisante des gels silicones aux films polyuréthanes pour les coussins et matelas de prévention des escarres ou pour les prothèses mammaires externes. Dans ces applications le gel silicone est complètement intégré dans une poche de film polyuréthane de telle façon qu'aucun contact n'est possible entre le gel et l'extérieur.

Le document US 2007/042108 A1 décrit des articles adhésifs, tel que des pansements, patches ou prothèses, comprenant un substrat plastique, une couche primaire d'accrochage constituée par example de méthylhydrogène cyclosiloxane et d'une couche formée par un gel silicone collant.

Lorsqu'au contraire des gels silicones collants sont utilisés comme adhésifs pour réaliser par exemple des bandes adhésives pour les pansements, les dispositifs de fixation de sacs de stomie, ou dans certaines applications d'habillement, les solutions de polymères du methylhydrogène siloxane permettent d'obtenir une adhésion satisfaisante des gels silicones aux films polyuréthanes mais le collant des gels est perdu ou fortement diminué, de telle façon que la bande adhésive ne présente plus la performance requise ce qui est un problème qui peut-être rédhibitoire pour ce type d'application.

Dans cet état de connaissance, l'un des objectifs essentiel de la présente invention est de fournir un article adhésif sur la peau ne présentant plus les problèmes décrits ci-dessus.

Cet objectif est atteint par l'invention qui concerne un article adhésif sur la peau comprenant:
(1) un substrat plastique S qui est de préférence un film plastique, revêtu sur au moins une des deux faces par:
(2) au moins une couche primaire d'accrochage C préparée par:
   a) application d'une composition constituée de:
      i) au moins un cyclosiloxane A, et
      ii) d'au moins un réticulant qui est soit une huile polyorganosiloxane **B** comprenant au moins un groupe alcényle lié à un atome de silicium et au moins un atome d'hydrogène lié à un atome de silicium, soit une résine polyorganosiloxane **F** comprenant au moins un motif siloxyle comprenant au moins un atome d'hydrogène lié à un atome de silicium,
   b) et séchage de ladite couche primaire d'accrochage **C** par évaporation du cyclosiloxane **A,** et
**(3)** au moins une couche **D** déposée sur la couche primaire d'accrochage **C,** ladite couche **D** étant constituée par un gel silicone **E.**

La Demanderesse a mis en oeuvre d'importants moyens de recherche et de nombreuses expérimentations pour atteindre cet objectif parmi d'autres. Et au terme de cela, elle a eu le mérite de trouver, de manière tout à fait surprenante et inattendue, qu'en utilisant dans la couche primaire d'accrochage et en lieu et place des réticulants classiques, tels que des polymères du methylhydrogène siloxane, l'huile polyorganosiloxane **B** ou la résine polyorganosiloxane **F** selon l'invention, alors une adhésion satisfaisante des gels silicones aux films supports plastiques de faible énergie de surface est obtenue tout en conservant ou améliorant le collant des gels, de sorte que l'article présente une excellente performance fonctionnelle.

De préférence, la résine polyorganosiloxane **F** est une résine choisie parmi le groupe constitué par : une résine M'Q où les atomes d'hydrogène liés au silicium sont inclus dans une partie des motifs siloxyles (M), une résine MD'Q où les atomes d'hydrogène liés au silicium sont inclus dans les motifs siloxyles (D), une résine MM'Q où les atomes d'hydrogène liés au silicium sont inclus dans une partie des motifs siloxyles (M), une résine MM'DQ où les atomes d'hydrogène liés au silicium sont inclus dans une partie des motifs siloxyles (M) et une résine MDT' où les atomes d'hydrogène liés au silicium sont inclus dans les motifs siloxyles (T), avec M, M', D, D', T' et Q étant des motifs siloxyles de formules suivantes :
M'= (H)(R₂)SiO_{1/2}
M = R₃SiO_{1/2}
D = R₂SiO_{2/2}
D' = (H)(R)SiO_{2/2}
T' = (H)SiO_{3/2}
Q = SiO_{4/2}
avec les radicaux R, identiques ou différents, représentant des groupes alkyle ou cycloalkyle en C₁-C₁₈.

Selon un mode de réalisation préféré, l'huile polyorganosiloxane **B** a pour formule:

**MDₓD'_{y}D^{Alk}_{z}M**

avec:
a) **M** = (R¹)₃SiO_{1/2}
   formule dans laquelle les radicaux R¹, identiques ou différents, correspondent soient à un groupement aryle, soient à un groupement alkyle en C₁-C₆ linéaire ou ramifié,
b) **D' =** (R²)(H)SiO_{2/2}
   formule dans laquelle le radical R² correspond soit à un groupement aryle, soit à un groupement alkyle, linéaire ou ramifié, en C₁-C₆, ;
c) **D** = (R³)₂SiO_{2/2}
   formule dans laquelle les radicaux R³ répondent à la même définition que R² décrit ci-dessus;
d) D^{Alk} = (R⁴)(X)SiO_{2/2}
   formule dans laquelle le radical R⁴ répond à la même définition que R² décrit ci-dessus; et le radical X correspond à un groupement alcényle ayant de 2 à 6 atomes de carbone et de préférence un groupement vinyle,
e) x est compris entre 0 et 200, de préférence entre 1 et 100 et encore plus préférentiellement entre 10 et 50,
f) y est compris entre 1 et 200, de préférence entre 10 et 100 et encore plus préférentiellement entre 40 et 80, et
g) z est compris entre 1 et 50, de préférence entre 2 et 20 et encore plus préférentiellement entre 2 et 10.

Selon un autre mode de réalisation, le substrat est un film plastique en polyuréthane ou en polyester.

De préférence, le gel silicone **E** est préparé par réticulation d'une composition silicone **K** comprenant :
- au moins un polyorganosiloxane **G** ayant en moyenne deux groupes alcényle liés à du silicium par molécule, lesdits groupes alcényle comptant chacun 2 à 6 atomes de carbone et aucun atome de silicium n'étant lié à plus d'un seul groupe alcényle,
- au moins un composé hydrogéné du silicium **H** ayant au moins 2 et de préférence au moins trois atomes d'hydrogène liés à du silicium par molécule,
- éventuellement au moins un polyorganosiloxane non fonctionnalisé **I** et
- un catalyseur d'hydrosylilation **J** à base de platine.

Les gels de silicone et autres gels sont couramment employés dans le domaine médical que ce soit à usage externe (prothèses mammaires, coussins ou matelas médicaux) ou à usage interne (prothèses mammaires placées in situ). Ils sont d'une très grande mobilité et ont de très bonnes propriétés mécaniques, leur densité étant proche de celle des tissus humains.

Pour toutes ces applications, les propriétés physiques de ces gels sont adaptées suivant l'utilisation en faisant varier les taux de motifs siloxyles porteurs de fonctions vinyle et SiH.

En général, le polyorganosiloxane **G** contient en moyenne deux groupes alcényle liés à du silicium par molécule, chaque groupe alcényle étant lié à un atome de silicium différent. Le polyorganosiloxane **G** est un polymère sensiblement linéaire, bien qu'un faible degré de ramification puisse exister. De préférence, les groupes alcényle sont fixés à des atomes de silicium qui sont éloignés entre eux dans la molécule, et au mieux, ils sont fixés aux atomes de silicium terminaux de la chaîne siloxane. Les groupes alcényle comptent au maximum 6 atomes de carbone et il peut s'agir par exemple de groupes vinyle, allyle ou hexényle, bien que ce soient de préférence des groupes vinyle. Les substituants organiques restants du polyorganosiloxane **G** sont choisis parmi les groupes alkyle et aryle, s'agissant de préférence de groupes alkyle n'ayant pas plus de 8 atomes de carbone et de groupes phényle. Des exemples de ces substituants restants sont les groupes méthyle, éthyle, propyle, isobutyle et phényle. Les plus volontiers mis en oeuvre sont des polydiméthylsiloxanes α,ω (diméthyvinylsiloxy) ou des polyorganosiloxanes de type poly(diméthylsiloxy) (méthylvinylsiloxy) α,ω (diméthylvinylsiloxy).

Le polyorganosiloxane **G** est un produit commercial comme par exemple les produits de la gamme des RHODORSIL^{®} 621 V de la société Bluestar Silicones, et sont largement divulgués tant en ce qui concerne leurs structures que leurs synthèses dans la littérature technique.

D'une manière préférée, le polyorganosiloxane **G** est sensiblement linéaire et possède une viscosité dynamique inférieure ou égale à 200 000 m.Pa.s, de préférence à 170000 mPa.s et encore plus préférentiellement comprise entre 20 et 165000 mPa.s.

Selon une autre variante, le % en poids de groupes réactifs alcényles liés directement à un atome de silicium, est compris entre 0,025 % et 3%.

Le composé hydrogéné du silicium **H** est en général un polyorganosiloxane, ou un silane, comportant au moins 2, de préférence 3, atomes d'hydrogène liés à du silicium par molécule. Ces atomes d'hydrogène peuvent être situés sur des motifs siloxanes terminaux et également sur des motifs siloxanes se trouvant dans la chaîne polymère, ou bien ils peuvent être situés uniquement à l'intérieur de la chaîne siloxane.

En pratique, les polyorganohydrogénosiloxanes **H** mis en oeuvre sont par exemple les polyorganosiloxanes de type poly(diméthylsiloxy)-(siloxyméthylhydrogéno)- α,ω-(diméthylhydrogénosiloxy) et les polydiméthylsiloxanes -α,ω-(diméthylhydrogénosiloxy). Ces POS (I) sont des produits commerciaux et sont largement divulgués tant en ce qui concerne leurs structures que leurs synthèses dans la littérature technique.

Pour les polyorganosiloxanes non fonctionnalisé **I,** les plus volontiers mis en oeuvre sont des huiles polydimethylsiloxanes-α,ω-(triméthylsiloxy) ou PDMS. Ces polyorganosiloxanes sont des produits commerciaux comme par exemple les produits de la gamme des RHODORSIL^{®} 47V (par exemple 47V50, 47V100, 47V500, 47V500, 47V12500 ou 47V30000) de la société Bluestar Silicones, et sont largement divulgués tant en ce qui concerne leurs structures que leurs synthèses dans la littérature technique.

D'une manière préférée, le polyorganosiloxanes non fonctionnalisé **I** est sensiblement linéaire et possède une viscosité dynamique inférieure ou égale à 50 000 mPa.s, de préférence comprise entre 20 et 40 000 mPa.s.

Le catalyseur **J** est un autre élément important de la composition pour former le gel. Il s'agit, de préférence, d'un complexe organométallique du platine ou bien encore de l'un des catalyseurs à base de platine traditionnellement mis en oeuvre pour la catalyse de ce type de réactions, par exemple, des groupes ≡SiH et des groupes ≡Si-Vinyle. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre autres. Le brevet US N° 2 823 218 divulgue un catalyseur d'hydrosilylation du type acide chloroplatinique et le brevet US N°3 419 593 est relatif à des catalyseurs formés par des complexes d'acide chloroplatinique et d'organosilicone du type vinylsiloxane. Des complexes de platine et d'hydrocarbures utiles comme catalyseur d'hydrosilylation sont divulgués par les brevets US N° 3 159 601 et 3 159 662. Le brevet US N° 3 723 497 décrit un acétylacétonate de platine et le brevet US N° 3 220 972 a pour objet des catalyseurs à base d'alcoolate de platine.

Pour le composant **J,** on entend par quantité efficace d'au moins un catalyseur de réaction d'hydrosilylation , la quantité suffisante pour amorcer la réaction d'hydrosilylation. Concernant la quantité catalytiquement efficace à mettre en oeuvre, il va de soi que l'homme du métier du domaine considéré est parfaitement à même de déterminer la quantité optimale de catalyseur pour promouvoir la réaction d'hydrosilylation . Cette quantité dépend notamment de la nature du catalyseur et des POS en cause. Pour fixer les idées on peut indiquer qu'elle sera comprise entre 0,001 et 0,5 % en poids par rapport au poids total de la composition.

De préférence, la quantité des constituants **G, H, I et J** est choisie de manière à ce que le rapport molaire r des atomes d'hydrogène liés au silicium aux radicaux alcényles (X) liés au silicium soit compris entre 0,5 :1 et 5 :1.

La composition silicone **K** peut en outre comprendre au moins un ralentisseur de la réaction d'addition ou un inhibiteur de réticulation choisi parmi les composés suivants :
- polyorganosiloxanes substitués par au moins un alcényle pouvant se présenter éventuellement sous forme cyclique, le tétraméthylvinyltétrasiloxane étant particulièrement préféré,
- la pyridine,
- les phosphines et les phosphites organiques,
- les amides insaturés,
- les maléates alkylés, et
- les alcools acétyléniques.

Ces alcools acétyléniques, (voir FR-A-1 528 464 et FR-A-2 372 874), qui font partie des bloqueurs thermiques de réaction d'hydrosilylation préférés, ont pour formule :

(R' )(R")(OH)C- C ≡ CH

formule dans laquelle,
- R' est un radical alkyle linéaire ou ramifié, ou un radical phényle ;
- R" est H ou un radical alkyle linéaire ou ramifié, ou un radical phényle ; les radicaux R', R" et l'atome de carbone situé en α de la triple liaison pouvant éventuellement former un cycle ; et
- le nombre total d'atomes de carbone contenu dans R' et R" étant d'au moins 5, de préférence de 9 à 20.

Lesdits alcools sont, de préférence, choisis parmi ceux présentant un point d'ébullition supérieur à 250°C. On peut citer à titre d'exemples :
- l'éthynyl-1-cyclohexanol-1 ;
- le méthyl-3 dodécyne-1 ol-3 ;
- le triméthyl-3,7,11 dodécyne-1 ol-3 ;
- le diphényl-1,1 propyne-2 ol-1 ;
- l'éthyl-3 éthyl-6 nonyne-1 ol-3 ;
- le méthyl-2 butyne-3 ol-2 ;
- le méthyl-3 pentadécyne-1 ol-3.

Ces alcools α-acétyléniques sont des produits du commerce. Un tel ralentisseur est présent à raison de 3 000 ppm au maximum, de préférence à raison de 100 à 1000 ppm par rapport au poids total des polyorganosiloxanes de la composition silicone.

Un autre objet de l'invention concerne un pansement ou patch à usage médical ou paramédical comprenant l'article adhésif sur la peau selon l'invention et tel que décrit ci-dessus.

S'agissant de la préparation du gel, on peut préciser que la réticulation de la composition en gel intervient à la température ambiante ou après chauffage à des températures comprises entre 50 et 200° C par exemple. Dans ce contexte, les durées de réticulation nécessaires sont, par exemple, comprises entre quelques mn et 1 heure 30 mn. Ces produits sont largement diffusés et décrits dans le commerce et sont bien connus de l'homme du métier.

Les exemples non limitatifs qui suivent, montrent diverses possibilités de formulation des compositions selon l'invention ainsi que les caractéristiques et les propriétés des gels silicones obtenus par réticulation desdites compositions.

### EXEMPLES

### Préparation du primaire d'adhérence selon l'invention

Le tableau ci-dessous décrit les formulations selon l'invention des primaires d'adhérence de gels silicones sur les films plastiques. Elles sont constituées de 90% poids du cyclosiloxane A et de 10% poids du réticulant.

| | Nature du cyclosiloxane **(A)** | Réticulant |
|---|---|---|
| Primaire A selon l'invention | Decamethylcyclopentasiloxane | Huile polyorganosiloxane **(B)** contenant des motifs Si-vinyle et des motifs Si-H |
| | | Silbione 20038 ® commercialisée par Bluestar Silicones |
| Primaire B selon l'invention | Decamethylcyclopentasiloxane | Résine M'Q **(F)** contenant des motifs Si-H. |
| | | Resin 10339 ® commercialisée par Bluestar Silicones |
| Primaire C comparatif PS 810 E | Décamethylcyclopentasiloxane | Huile polyorganosiloxane contenant des motifs SiH (polymère du méthyl hydrogène siloxane) |

### Mode opératoire :

2 grammes du réticulant sont dilués dans 18 g de décaméthylcyclopentasiloxane. Le mélange est agité pendant 5 minutes à température ambiante. Le substrat plastique est un film polyester d'une épaisseur d'environ 70µm. Le primaire est appliqué sur le film avec une brosse constituant une couche primaire d'accrochage d'environ 40 à 50 g/cm² puis séché pendant 30 minutes à 60°C.

Ensuite, le RT Gel 4320 ® commercialisée par Bluestar Silicones est appliqué avec un couteau sur la couche primaire d'accrochage et l'ensemble est séché dans un four pendant 15 minutes à 120°C.

### Test réalisés

Deux paramètres ont été mesurés sur les échantillons ainsi obtenus.
- l'adhésion du gel silicone sur le film polyester mesuré par un test de pelage du gel silicone au doigt (rubbing test).
- le tack ou « effet collant » du gel silicone via le Probe Tack Device (PT-1000). Un tack ou effet collant de 4 mJ/cm² est jugé satisfaisant pour l'application.

Les résultats obtenus sont présentés dans le tableau suivant :

| **Echantillon** | Adhésion du gel silicone au substrat plastique | Probe Tack (mJ/cm²) |
|---|---|---|
| Substrat plastique avec le primaire A selon l'invention | Très bonne | 6,11 |
| Substrat plastique avec le primaire B selon l'invention | Très bonne | 4,04 |
| Substrat plastique avec le primaire C comparatif | Très bonne | 0,42 |

Ces résultats montrent que les primaires selon l'invention permettent de maintenir un bon niveau de collant pour les gels tout en obtenant une excellente adhésion du gel silicone au film polyester.

## Revendications

1. Article adhésif sur la peau comprenant:
**(1)** un substrat plastique **S** qui est de préférence un film plastique, revêtu sur au moins une des deux faces par:
**(2)** au moins une couche primaire d'accrochage **C** préparée par:
a) application d'une composition constituée de:
i) au moins un cyclosiloxane **A,** et
ii) d'au moins un réticulant qui est soit une huile polyorganosiloxane **B** comprenant au moins un groupe alcényle lié à un atome de silicium et au moins un atome d'hydrogène lié à un atome de silicium, soit une résine polyorganosiloxane **F** comprenant au moins un motif siloxyle comprenant au moins un atome d'hydrogène lié à un atome de silicium,
b) et séchage de ladite couche primaire d'accrochage **C** par évaporation du cyclosiloxane **A,** et
**(3)** au moins une couche **D** déposée sur la couche primaire d'accrochage **C,** ladite couche **D** étant constituée par un gel silicone **E.**

2. Article selon la revendication 1 **caractérisé en ce que** la résine polyorganosiloxane **F** est une résine choisie parmi le groupe constitué par : une résine M'Q où les atomes d'hydrogène liés au silicium sont inclus dans une partie des motifs siloxyles (M), une résine MD'Q où les atomes d'hydrogène liés au silicium sont inclus dans les motifs siloxyles (D), une résine MM'Q où les atomes d'hydrogène liés au silicium sont inclus dans une partie des motifs siloxyles (M), une résine MM'DQ où les atomes d'hydrogène liés au silicium sont inclus dans une partie des motifs siloxyles (M) et une résine MDT' où les atomes d'hydrogène liés au silicium sont inclus dans les motifs siloxyles (T), avec M, M', D, D', T' et Q étant des motifs siloxyles de formules suivantes :
M'= (H)(R₂)SiO_{1/2}
M = R₃SiO_{1/2}
D = R₂SiO_{2/2}
D' = (H)(R)SiO_{2/2}
T' = (H)SiO_{3/2}
Q = SiO_{4/2}
avec les radicaux R, identiques ou différents, représentant des groupes alkyle ou cycloalkyle en C₁-C₁₈.

3. Article selon la revendication 1 dans lequel l'huile polyorganosiloxane **B** a pour formule :
**MDₓD'_{y}D^{Alk}_{z}M**
avec:
a) **M** = (R¹)₃SiO_{1/2}
formule dans laquelle les radicaux R¹, identiques ou différents, correspondent soient à un groupement aryle, soient à un groupement alkyle en C₁-C₆ linéaire ou ramifié,
b) **D' =** (R²)(H)SiO_{2/2}
formule dans laquelle le radical R² correspond soit à un groupement aryle, soit à un groupement alkyle, linéaire ou ramifié, en C₁-C₆,;
c) **D** = (R³)₂SiO_{2/2}
formule dans laquelle les radicaux R³ répondent à la même définition que R² décrit ci-dessus;
d) **D^{Alk}** = (R⁴)(X)SiO_{2/2}
formule dans laquelle le radical R⁴ répond à la même définition que R² décrit ci-dessus; et le radical X correspond à un groupement alcényle ayant de 2 à 6 atomes de carbone et de préférence un groupement vinyle,
e) x est compris entre 0 et 200, de préférence entre 1 et 100 et encore plus préférentiellement entre 10 et 50,
f) y est compris entre 1 et 200, de préférence entre 10 et 100 et encore plus préférentiellement entre 40 et 80, et
g) z est compris entre 1 et 50, de préférence entre 2 et 20 et encore plus préférentiellement entre 2 et 10.

4. Article selon la revendication 1 dans lequel le substrat est un film plastique en polyuréthane ou en polyester.

5. Article selon la revendication 1 dans lequel le gel silicone **E** est préparé par réticulation d'une composition silicone comprenant :
- au moins un polyorganosiloxane **G** ayant en moyenne deux groupes alcényle liés à du silicium par molécule, lesdits groupes alcényle comptant chacun 2 à 6 atomes de carbone et aucun atome de silicium n'étant lié à plus d'un seul groupe alcényle,
- au moins un composé hydrogéné du silicium **H** ayant au moins 2 et de préférence au moins trois atomes d'hydrogène liés à du silicium par molécule,
- éventuellement au moins un polyorganosiloxane non fonctionnalisé **I** et
- un catalyseur d'hydrosylilation **J** à base de platine.

6. Pansement ou patch à usage médical ou paramédical comprenant l'article adhésif sur la peau et tel que décrit selon l'une quelconque des revendications précédentes.

## Patentansprüche

1. Hautklebeartikel, umfassend:
(1) ein Kunststoffsubstrat **S,** vorzugsweise eine Kunststofffolie, auf mindestens einer der beiden Seiten beschichtet mit:
(2) mindestens eine Haftgrundierungsschicht **C,** hergestellt durch
a) Aufbringen einer Zusammensetzung, bestehend aus:
i) mindestens einem Cyclosiloxan **A** und
ii) mindestens einem Vernetzer, bei dem es sich entweder um ein Polyorganosiloxanöl **B** mit mindestens einer an ein Siliciumatom gebundenen Alkenylgruppe und mindestens einem an ein Siliciumatom gebundenen Wasserstoffatom oder einen Polyorganosiloxanharz **F** mit mindestens einer Siloxyleinheit mit mindestens einem an ein Siliciumatom gebundenen Wasserstoffatom handelt,
b) und Trocknen der Haftgrundierungsschicht **C** durch Verdampfen des Cyclosiloxans **A,** und
(3) mindestens eine auf der Haftgrundierungsschicht **C** aufgebrachte Schicht **D,** wobei die Schicht **D** aus einem Silikongel E besteht.

2. Artikel nach Anspruch 1, **dadurch gekennzeichnet, dass** es sich bei dem Polyorganosiloxanharz **F** um ein Harz aus der Gruppe bestehend aus: einem M'Q-Harz, wobei die an das Silicium gebundenen Wasserstoffatome in einem Teil der Siloxyleinheiten (M) enthalten sind, einem MD'Q-Harz, wobei die an das Silicium gebundenen Wasserstoffatome in den Siloxyleinheiten (D) enthalten sind, einem MM'Q-Harz, wobei die an das Silicium gebundenen Wasserstoffatome in einem Teil der Siloxyleinheiten (M) enthalten sind, einem MM'DQ-Harz, wobei die an das Silicium gebundenen Wasserstoffatome in einem Teil der Siloxyleinheiten (M) enthalten sind, und einem MDT'-Harz, wobei die an das Silicium gebundenen Wasserstoffatome in den Siloxyleinheiten (T) enthalten sind, wobei M, M', D, D', T' und Q Siloxyleinheiten der folgenden Formeln sind:
M' = (H) (R₂)SiO_{1/2}
M = R₃SiO_{1/2}
D = R₂SiO_{2/2}
D' = (H)(R)SiO_{2/2}
T' = (H)SiO_{3/2}
Q = SiO_{4/2}
wobei die Reste R gleich oder verschieden sind und für C₁-C₁₈-Alkyl- oder -Cycloalkylgruppen stehen.

3. Artikel nach Anspruch 1, wobei das Polyorganosiloxanöl **B** die folgende Formel aufweist:
**MDₓD'_{y}D^{Alk}_{z}M**
wobei:
a) **M** = (R¹)₃SiO_{1/2}
worin die Reste R¹ gleich oder verschieden sind und entweder einer Arylgruppe oder einer linearen oder verzweigten C₁-C₆-Alkylgruppe entsprechen;
b) **D' =** (R²)(H)SiO_{2/2}
worin der Rest R² entweder eine Arylgruppe oder einer linearen oder verzweigten C₁-C₆-Alkylgruppe entspricht;
c) **D** = (R³)₂SiO_{2/2}
worin die Reste R³ der gleichen Definition wie der oben beschriebene Rest R² entsprechen;
d) **D^{Alk}** = (R⁴) (X)SiO_{2/2}
worin der Rest R⁴ der gleichen Definition wie der oben beschriebene Rest R² entspricht und der Rest X einer Alkenylgruppe mit 2 bis 6 Kohlenstoffatomen und vorzugsweise einer Vinylgruppe entspricht,
e) x zwischen 0 und 200, vorzugsweise zwischen 1 und 100 und noch weiter bevorzugt zwischen 10 und 50 liegt,
f) y zwischen 1 und 200, vorzugsweise zwischen 10 und 100 und noch weiter bevorzugt zwischen 40 und 80 liegt und
g) z zwischen 1 und 50, vorzugsweise zwischen 2 und 20 und noch weiter bevorzugt zwischen 2 und 10 liegt.

4. Artikel nach Anspruch 1, wobei es sich bei dem Substrat um eine Kunststofffolie aus Polyurethan oder aus Polyester handelt.

5. Artikel nach Anspruch 1, wobei das Silikongel **E** durch Vernetzung einer Silikonzusammensetzung, die
- mindestens ein Polyorganosiloxan **G** mit durchschnittlich zwei siliciumgebundenen Alkenylgruppen pro Molekül, wobei die Alkenylgruppen jeweils 2 bis 6 Kohlenstoffatome und kein Siliciumatom, das an mehr als eine Alkenylgruppe gebunden ist, enthalten,
- mindestens eine wasserstoffhaltige Siliciumverbindung H mit mindestens 2 und vorzugsweise mindestens drei siliciumgebundenen Wasserstoffatomen pro Molekül,
- gegebenenfalls mindestens ein nicht funktionalisiertes Polyorganosiloxan **I** und
- einen Hydrosilylierungskatalysator **J** auf Basis von Platin
umfasst, hergestellt wird.

6. Verband oder Pflaster zur medizinischen oder paramedizinischen Verwendung, umfassend einen Hautklebeartikel gemäß einem der vorhergehenden Ansprüche.

## Claims

1. Skin-adhesive article comprising:
**(1)** a plastics substrate **S** that is preferably a plastics film, coated on at least one of the two faces with:
**(2)** at least one adhesion primer layer **C** prepared by:
a) applying a composition composed of:
i) at least one cyclosiloxane **A,** and
ii) at least one crosslinker which is either a polyorganosiloxane oil **B** comprising at least one alkenyl group bonded to a silicon atom and at least one hydrogen atom bonded to a silicon atom, or a polyorganosiloxane resin **F** comprising at least one siloxyl unit comprising at least one hydrogen atom bonded to a silicon atom,
b) and drying said adhesion primer layer **C** by evaporating the cyclosiloxane **A,** and
(3) at least one layer **D** applied to the adhesion primer layer **C,** said layer **D** consisting of a silicone gel **E.**

2. Article according to Claim 1, **characterized in that** the polyorganosiloxane resin **F** is a resin selected from the group consisting of the following: a resin M'Q in which the hydrogen atoms bonded to the silicon are included in part of the siloxyl units (M), a resin MD'Q in which the hydrogen atoms bonded to the silicon are included in the siloxyl units (D), a resin MM'Q in which the hydrogen atoms bonded to the silicon are included in part of the siloxyl units (M), a resin MM'DQ in which the hydrogen atoms bonded to the silicon are included in part of the siloxyl units (M), and a resin MDT' in which the hydrogen atoms bonded to the silicon are included in the siloxyl units (T), where M, M', D, D', T', and Q are siloxyl units of formulae below:
M' = (H)(R₂)SiO_{1/2}
M = R₃SiO_{1/2}
D = R₂SiO_{2/2}
D' = (H)(R)SiO_{2/2}
T' = (H)SiO_{2/2}
Q = SiO_{4/2}
where the identical or different radicals R represent C₁-C₁₈ cycloalkyl or alkyl groups.

3. Article according to Claim 1, wherein the formula of the polyorganosiloxane oil **B** is as follows:
**MDₓD'_{y}D^{Alk}_{z}M**
where:
a) **M =** (R¹)₃SiO_{1/2}
in which formula the identical or different radicals R¹ correspond either to an aryl group or to a linear or branched C₁-C₆ alkyl group,
b) **D' =** (R²)(H)SiO_{2/2}
in which formula the radical R² corresponds either to an aryl group or to a linear or branched C₁-C₆ alkyl group,
c) **D** = (R³)₂SiO_{2/2}
in which formula the radicals R³ have the same definition as R² described above,
d) **D^{Al-k} =** (R⁴)(X)SiO_{2/2}
in which formula the radical R⁴ has the same definition as R² described above, and the radical X corresponds to an alkenyl group having from 2 to 6 carbon atoms, and preferably a vinyl group,
e) x is between 0 and 200, preferably between 1 and 100, and more preferably between 10 and 50,
f) y is between 1 and 200, preferably between 10 and 100, and more preferably between 40 and 80, and
g) z is between 1 and 50, preferably between 2 and 20, and more preferably between 2 and 10.

4. Article according to Claim 1, wherein the substrate is a plastics film made of polyurethane or of polyester.

5. Article according to Claim 1, wherein the silicone gel E is prepared by crosslinking a silicone composition comprising:
- at least one polyorganosiloxane **G** having on average two alkenyl groups bonded to silicon per molecule, said alkenyl groups each containing 2 to 6 carbon atoms, and no silicon atom being bonded to more than one single alkenyl group,
- at least one hydrogen-bearing silicon compound **H** having at least two and preferably at least three hydrogen atoms bonded to silicon per molecule,
- optionally at least one nonfunctionalized polyorganosiloxane **I,** and
- a platinum-based hydrosilylation catalyst **J.**

6. Dressing or patch for medical or paramedical use, comprising the skin-adhesive article as described in any of the preceding claims.
